(19) Europäisches Patentamt
European Patent Office
Office européen des brevets

(11) Publication number: **0 101 034 B1**

(12) **EUROPEAN PATENT SPECIFICATION**

(45) Date of publication of patent specification: **22.10.86**

(51) Int. Cl.$^4$: **C 07 D 261/14**

(21) Application number: **83107773.0**

(22) Date of filing: **06.08.83**

(54) **Method for preparing 3-amino-5-(substituted)isoxazole.**

(30) Priority: **09.08.82 US 406593**

(43) Date of publication of application:
**22.02.84 Bulletin 84/08**

(45) Publication of the grant of the patent:
**22.10.86 Bulletin 86/43**

(84) Designated Contracting States:
**BE DE FR GB LU NL SE**

(56) References cited:
**EP-A-0 042 732**
**AT-B- 365 583**
**DE-A-2 825 194**

(73) Proprietor: **PPG INDUSTRIES, INC.**
**One PPG Place**
**Pittsburgh Pennsylvania 15272 (US)**

(72) Inventor: **Govindan, Cheruthuruthil K.**
**1163 Clark Road**
**Wadsworth Ohio 44281 (US)**

(74) Representative: **Hann, Michael, Dr. et al**
**Patentanwälte Dr. Michael Hann Dr. H.-G.**
**Sternagel Sander Aue 30**
**D-5060 Bergisch Gladbach 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 101 034 B1

**Description**

This invention relates to a method for preparing 3-amino-5-(substituted)isoxazole.

3-amino-5-(substituted)isoxazoles, e.g., 3-amino-5-(*t*-butyl)isoxazole, are valuable intermediates used in the production of herbicidally active isoxazole derivatives.

DE—A—2825194 discloses the preparation of 3-amino-5-(substituted)isoxazoles from β-ketonitril and $NH_2OH$ via a iminoether and works in the absence of water.

AT—B—365 583 shows the preparation of 3-amino-5-(*t*-butyl)isoxazole by reacting hydroxyl amine or a salt thereof with pivaloyl acetonitrile in an aqueous medium at a pH range of from 5 to 8, preferably from 6.0 to 7.0. This process does not comprise an extraction of the initial reaction product with a water immiscible solvent.

This invention provides a method for preparing 3-amino-5-(substituted)isoxazole represented by the Formula (I):

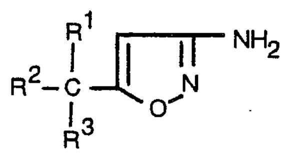

(I)

wherein

$R^1$ is hydrogen, $C_1$ to $C_4$ alkyl or up to $C_6$ cycloalkyl;

$R^2$ is $C_1$ to $C_4$ alkyl, up to $C_6$ cycloalkyl; and

$R^3$ is $C_1$ to $C_4$ alkyl.

The method of this invention has been found particularly suitable for preparing 3-amino-5-(*t*-butyl)isoxazole, i.e., a compound of Formula (I) wherein $R^1$, $R^2$ and $R^3$ are methyl; but, it is believed that any compound within the scope of Formula (I) could be prepared by the method of this invention.

More particularly, in the first step of the method of this invention, an appropriately substituted acetonitrile of the Formula (II):

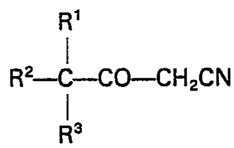

(II)

wherein $R^1$, $R^2$ and $R^3$ are as previously defined, is reacted with hydroxylamine or a salt thereof, in water, at a pH in the range of 8.3 to 8.7, preferably at pH 8.4 to 8.5, at a temperature ranging from ambient to 60°C, preferably at a temperature in the range of from 50°C to 60°C. At least stoichimetrically equivalent amounts of reactants are typically used and the hydroxylamine can be used as such or can be used in its salt form such as hydroxylamine hydrochloride or hydroxylamine sulfate. The reaction time will, of course, vary inversely with temperature and the desired degree of completeness of reaction. At, for example, 50°C, the reaction is typically substantially complete in 10 to 20 hours. The pH of the reaction mixture can be adjusted with any suitable organic or inorganic base, such as, for example, ammonia, triethylamine, alkali metal hydroxides, such as sodium, potassium or lithium hydroxide, alkali metal carbonate, such as sodium or potassium carbonate. An inorganic base is preferably used.

Under the aforesaid conditions of pH and temperature at which the Formula (II) acetonitrile and hydroxylamine are reacted, it has been observed that the reaction product consists largely of a mixture of amidoxime of the Formula (III):

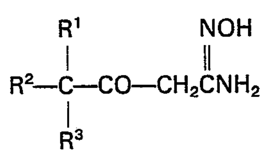

(III)

and from 7 to 10 weight percent of 5-amino-3-(substituted)isoxazole of the Formula (IV):

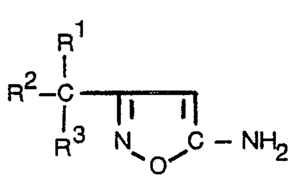

(IV)

wherein $R^1$, $R^2$, $R^3$ in each of the Formulae (III) and (IV) compounds are as previously defined. The Formula (III) amidoxime is the precursor for the desired Formula (I) compound, which latter is prepared by acid hydrolysis of the former. To prevent formation of undesirable by-products, for example, the 3-[3-(substituted)-5-isoxazolyl]amino-5-(substituted)isoxazole dimer, it has been found that a major portion of

the Formula (IV) compound as well as trace amounts of other impurities can be readily and selectively removed by liquid-liquid extraction.

Consequently, in the second step of the process of this invention, the aqueous reaction mixture from the first step is extracted with a water immiscible organic solvent or mixture of solvents. Any water immiscible organic solvent or solvent mixture can be used, for example, benzene, toluene, carbon tetrachloride, chloroform, dichloroethane, or a mixture of such solvents, e.g., carbon tetrachloride and chloroform, hexane and chloroform, carbon tetrachloride and dichloroethane.

After solvent extraction and phase separation, the aqueous phase containing the Formula (III) amidoxime is in the third step acidified to a pH in the range of 5 to 5.5 and heated to a temperature in the range of 40°C to 60°C, preferably 50°C to 55°C, for a time sufficient to cyclicize the Formula (IV) aldoxime to the Formula (I) product.

The quantity of acid utilized in order to adjust the pH of the said aqueous phase could be, at most, about 0.1 mole equivalent in excess of that required to arrive at a neutral pH of 7.0; and typically the amount of acid required would be less than 0.05 mole equivalent. Preferably the acid used to adjust the pH to within the desired range is a mineral acid, such as, hydrochloric, sulfuric or phosphoric acid, although organic acids may be used with equal efficacy.

In the fourth step of the process of this invention, the precipitation of the desired Formula (I) product is completed by adjusting the pH of the reaction mixture to at least 10 and preferably in the range of 10 to 11 by addition of one or more of the aforesaid organic and preferably inorganic bases. The product may be separated from the aqueous alkaline reaction medium by any technique known to the art, such as, for example, filtration, centrifugation or the like.

The process of the invention provides a straightforward means of preparing high purity 3-amino-5-(substituted)isoxazole in high yield devoid of the disadvantages of the prior art. It might also be added that the process of the invention also permits recovery of 5-amino-3-(substituted)isoxazole from the organic solvent in which the same was selectively extracted.

To summarize the method of this invention, the same involves the steps of:

(a) reacting, in water at a temperature in the range of from ambient to 60°C an appropriately substituted acetonitrile of the Formula (II) with hydroxylamine or a salt thereof at a basic pH in the range of from 8.3 to 8.7, preferably pH 8.4 to 8.5;

(b) extracting the reaction mixture with a water immiscible organic solvent or solvent mixture;

(c) acidifying the aqueous phase from step (b) to a acidic pH in the range of 5.0 to 5.5; and heating the aqueous phase from step (b) to a temperature in the range of 40° to 60°C;

(d) adjusting the pH of the reaction mixture of step (c) to a alkaline pH of at least 10, preferably pH 10 to 11; and

(e) isolating the precipitated product, which is 3-amino-5-(substituted)isoxazole.

It will be further understood that 5-amino-3-(substituted)isoxazole removed from the reaction mixture by extraction in step (b) can be conveniently isolated by removal of solvent, for example, evaporation and further purified by recrystallization, which 5-amino-3-(substituted)isoxazole also is useful as an intermediate in, for example, the preparation of certain herbicidally active compounds.

The method of this invention is illustrated by the following Example which describes the preparation of a preferred 3-(amino)-5-(substituted)isoxazole, namely, 3-amino-5-(*t*-butyl)isoxazole.

## Example

A. To a suitably sized flask, provided with a mechanical stirrer and a thermometer was charged 22.5 grams of sodium hydroxide, 720 milliliters of water, 62.5 grams of pivaloyl acetonitrile and 45.1 grams of hydroxylamine sulfate. The reaction mixture, having a pH of 8.43 was heated for 10 hours at 50°C, allowed to cool to ambient (20°C) temperature and permitted to react at ambient temperature for an additional 8 hours. The reaction mixture was divided into two equal portions and one portion was extracted with a first portion of 75 milliliters of carbon tetrachloride and a second portion of 25 milliliters of carbon tetrachloride.

Following phase separation, the aqueous phase was acidified by addition of 10 milliliters of concentrated hydrochloric acid to pH to 5.5 and permitted to react, with stirring, at 50°C for about 2-1/2 hours. After cooling the pH of the reaction mixture was adjusted to pH 10—11 by addition of 50 percent aqueous sodium hydroxide solution. The resulting precipate was filtered, washed and dried, affording 27.8 grams (79.4 percent yield) of 98.2 percent assay, 3-amino-5-(*t*-butyl)isoxazole melting at 108—111°C.

The organic, i.e., carbon tetrachloride, extracts were combined, dried and concentrated on a rotary evaporator, yielding 2.49 grams of residue identified by HPLC as consisting mainly of 5-amino-3-(*t*-butyl)isoxazole.

B. The second portion of the pivaloyl acetonitrilehydroxylamine sulfate reaction mixture was adjusted to pH 11.63 by addition of 2 milliliters of 50 percent aqueous sodium hydroxide solution and extracted with 100 milliliters of carbontetrachloride.

Following phase separation, the organic layer was dried and concentrated on a rotary evaporator yielding 2.25 grams of residue identified by HPLC as consisting mainly of 5-amino-3-(*t*-butyl)isoxazole.

The aqueous phase was acidified by addition of 12 milliliters of concentrated hydrochloric acid to pH 5 to 5.5, and permitted to react with stirring at 50°C for about 2 hours. After cooling, the pH of the reaction mixture was adjusted to pH 11.5 by addition of 50 percent aqueous sodium hydroxide solution. The

resulting precipitate was filtered, washed and dried affording 27.0 grams (77 percent yield) of 98 percent assay 3-amino-5-(*t*-butyl)isoxazole melting at 108—110°C.

It should be noted that the experiment described in part B of this Example was performed to determine whether elevating the pH after completion of the reaction between the pivaloyl acetonitrile and hydroxylamine sulfate might have a favorable effect on the solubility of the amidoxime intermediate and the yield and purity of the 3-amino-5-(*t*-butyl)isoxazole product. As the results indicate, this pH adjustment had no significant effect on yield or purity of 3-amino-5-(*t*-butyl)isoxazole product.

Although a specific embodiment of the process of this invention has been described by the foregoing Example, it will be appreciated that many variations may be made therein, which variations would be apparent to one skilled in the art, since the said variations involve only the choice of substituted acetonitrile starting material, the choice of acid and base with which to effect the requisite pH adjustments and the choice of suitable water immiscible organic solvent or solvent mixture.

**Claims**

1. A process for preparing 3-amino-5-(substituted)isoxazole of the formula (I)

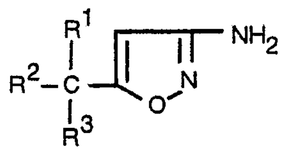

(I)

wherein

$R^1$ is hydrogen, $C_1$ to $C_4$ alkyl or up to $C_6$ cycloalkyl;
$R^2$ is $C_1$ to $C_4$ alkyl, up to $C_6$ cycloalkyl; and
$R^3$ is $C_1$ to $C_4$ alkyl.

by reacting in water under controlled pH conditions hydroxylamine, or a salt thereof with a substituted acetonitrile, characterized by the steps

(a) reacting, in water, at a temperature in the range of from ambient to 60°C and a pH in the range of from 8.3 to 8.7, hydroxylamine or a salt thereof with a substituted acetonitrile of the formula (II)

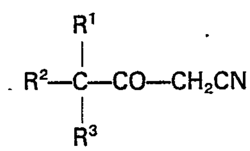

(II)

wherein $R^1$, $R^2$ and $R^3$ are as previously defined;

(b) extracting the reaction mixture from step (a) with a water immiscible organic solvent or mixture of solvents,

(c) adjusting the pH of the aqueous phase from step (b) in the range of 5 to 5.5 and heating the aqueous phase from step (b) to a temperature in the range of 40° to 60°C,

(d) adjusting the pH of the acidified aqueous phase of step (c) to a pH of at least 10; and

(e) isolating the precipitated 3-amino-5-(substituted)isoxazole product from the reaction mixture of step (d).

2. The process of Claim 1 wherein the substituted acetonitrile is pivaloyl acetonitrile and the 3-amino-5-(substituted)isoxazole product is 3-amino-5-(*t*-butyl)isoxazole.

3. The process of Claim 1 wherein the reaction temperature in step (a) is in the range of from 50°C to 55°C.

4. The process of Claim 1 wherein the pH of the reaction mixture in step (a) is in the range of from 8.3 to 8.4.

5. The process of Claim 1 wherein the pH of the reaction mixture in step (d) is in the range of from 10 to 11.

6. The process of Claim 2 wherein the reaction temperature in step (a) is in the range of from 50°C to 60°C.

7. The process of Claim 6 wherein the reaction mixture in step (a) is at a pH of 8.4 to 8.5.

8. The process of Claim 6 wherein the reaction mixture in step (d) is at a pH in the range of from 10 to 11.

**Patentansprüche**

1. Verfahren zum Herstellen von 3-Amino-5-(substituierten)isoxazol der Formel (I)

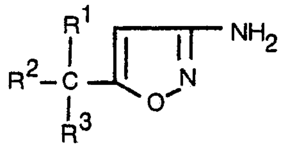

(I)

in der $R^1$ Wasserstoff, $C_1$ bis $C_4$ Alkyl oder bis zu $C_6$ Cycloalkyl ist, $R^2$ $C_1$ bis $C_4$ Alkyl, bis zu $C_6$ Cycloalkyl ist und $R^3$ $C_1$ bis $C_4$ Alkyl ist, durch Umsetzen von Hydroxylamin oder einem seiner Salze mit einem substituierten Acetonitril in Wasser unter gesteuerten pH-Bedingungen, gekennzeichnet durch die Schritte

a) Umsetzen in Wasser bei einer Temperatur im Bereich von Umgebungstemperatur bis 60°C und einem pH im Bereich von 8,3 bis 8,7 von Hydroxylamin oder einem seiner Salze mit einem substituierten Acetonitril der Formel (II)

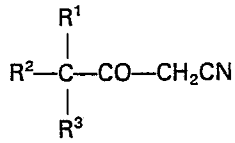

(II)

in der $R^1$, $R^2$ und $R^3$ die zuvor angegebene Bedeutung haben,

b) Extrahieren der Reaktionsmischung des Schrittes (a) mit einem mit Wasser nicht mischbaren organischen Lösungsmittel oder einer Mischung von Lösungsmitteln,

c) Einstellen des pH-Wertes der wässrigen Phase von Schritt (b) auf den Bereich von 5 bis 5,5 und Erwärmen der wässrigen Phase von Schritt (b) auf eine Temperatur im Bereich von 40 bis 60°C,

d) Einstellen des pH der angesäuerten wässrigen Phase des Schrittes (c) auf einen pH von mindestens 10 und

e) Isolieren des ausgefällten 3-Amino-5-(substituierten)isoxazol-Produktes aus der Reaktionsmischung des Schrittes (d).

2. Verfahren nach Anspruch 1, dadurch gekennzeichnet, daß das substituierte Acetonitril Pivaloylacetonitril ist und das 3-Amino-5-(substituierte)isoxazol-Produkt 3-Amino-5-(*t*-butyl)isoxazol ist.

3. Verfahren nach Anspruch 1, dadurch gekennzeichnet, daß die Reaktionstemperatur in Schritt (a) im Bereich von 50 bis 55°C beträgt.

4. Verfahren nach Anspruch 1, dadurch gekennzeichnet, daß der pH der Reaktionsmischung in Schritt (a) im Bereich von 8,3 bis 8,4 liegt.

5. Verfahren nach Anspruch 1, dadurch gekennzeichnet, daß der pH der Reaktionsmischung in Schritt (d) im Bereich von 10 bis 11 liegt.

6. Verfahren nach Anspruch 2, dadurch gekennzeichnet, daß die Reaktionstemperatur in Schritt (a) im Bereich von 50 bis 60°C beträgt.

7. Verfahren nach Anspruch 6, dadurch gekennzeichnet, daß die Reaktionsmischung in Stufe (a) einen pH-Wert von 8,4 bis 8,5 aufweist.

8. Verfahren nach Anspruch 6, dadurch gekennzeichnet, daß die Reaktionsmischung in Schritt (d) einen pH-Wert im Bereich von 10 bis 11 aufweist.

**Revendications**

1. Procédé de préparation d'un amino-3 isoxazole substitué en position 5, représenté par la Formule (I)

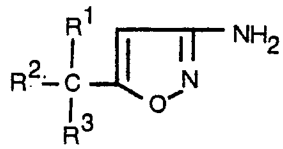

(I)

formule dans laquelle:

— $R^1$ est l'hydrogène, un reste alkyle en $C_1$—$C_4$ ou un reste cycloalkyle allant jusqu'en $C_6$;

— $R^2$ est un reste alkyle en $C_1$—$C_4$, un reste cycloalkyle allant jusqu'en $C_6$; et

— $R^3$ est un reste alkyle en $C_1$—$C_4$;

par réaction, dans l'eau, dans des conditions de pH contrôlé, de l'hydroxylamine, ou un sel de cette dernière, avec un acétonitrile substitué, caractérisé par le fait qu'il comprend les étapes consistant à:

(a) faire réagir, dans l'eau, à une température se situant dans la plage allant de la température ambiante à 60°C et à un pH se situant dans la plage de 8,3 à 8,7, de l'hydroxylamine, ou un sel de cette dernière, avec un acétonitrile substitué représenté par le Formule (II):

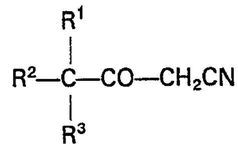 (II)

formule dans laquelle $R^1$, $R^2$ et $R^3$ sont tels que précédemment définis;

(b) extraire le mélange réactionnel provenant de l'étape (a) par un solvant organique non miscible à l'eau ou un mélange de solvants organiques non miscibles à l'eau,

(c) ajuster le pH de la phase aqueuse provenant de l'étape (b) dans la plage de 5 à 5,5 et chauffer la phase aqueuse provenant de l'étape (b) à une température se situant dans la plage de 40°C à 60°C,

(d) ajuster le pH de la phase aqueuse acidifée provenant de l'étape (c) à un pH d'au moins 10; et

(e) isoler le produit précipité consistant en amino-3 isoxazole substitué en position 5, à partir du mélange réactionnel provenant de l'étape (d).

2. Procédé selon la revendication 1, dans lequel l'acétonitrile substitué est le pivaloyl acétonitrile et l'amino-3 isoxazole substitué en position 5 qui est produit est l'amino-3 (*t*-butyl)-5 isoxazole.

3. Procédé selon la revendication 1, dans lequel la température de réaction à l'étape (a) se situe dans la plage allant de 50°C à 55°C.

4. Procédé selon la revendication 1, dans lequel le pH du mélange réactionnel à l'étape (a) se situe dans la plage allant de 8,3 à 8,4.

5. Procédé selon la revendication 1, dans lequel le pH du mélange réactionnel à l'étape (d) se situe dans la plage allant de 10 à 11.

6. Procédé selon la revendication 2, dans lequel la température de réaction à l'étape (2) se situe dans la plage allant de 50°C à 60°C.

7. Procédé selon la revendication 6, dans lequel le mélange réactionnel à l'étape (a) est à un pH de 8,4 à 8,5.

8. Procédé selon la revendication 6, dans lequel le mélange réactionnel à l'étape (d) est à un pH se situant dans la plage allant de 10 à 11.